# EUROPEAN PATENT APPLICATION

(11) **EP 1 001 022 A1**
(43) Date of publication of application: **17.05.2000**
(21) Application number: 98119583.7
(22) Date of filing: 16.10.1998
(51) Int. Cl.: C12N 15/12, C07K 14/47, A61K 38/17

(54) **CAMEL, an alternative translation product of the tumour antigen LAGE-1**

(71) Applicant: Boehringer Ingelheim International GmbH, 55216 Ingelheim (DE); University Hospital Leiden, 2321 RP, Leiden (NL)
(72) Inventor: Schrier, Peter, 2313 ZT Leiden (NL); Aarnoudse, Corlien A., 2224 XM Katwijk (NL)
(74) Representative: Laudien, Dieter, Dr.

(57) **Abstract**

Tumor-associated antigens and DNAs encoding them. The tumor-associated antigens are encoded by an open reading frame of the LAGE-1 gene. The tumor-associated antigens, immunogenic (poly) peptides derived therefrom and DNAs encoding them, are useful for cancer immunotherapy.

## Description

The present invention relates to the field of cancer therapy, more specifically to tumor-associated antigens.

Cytotoxic T lymphocytes (CTL₅) play an important role in the defense against melanoma. Melanoma-specific CTL clones have been obtained from either tumor infiltrating lymphocytes (TIL) *in vitro* stimulated with cytokines, or from peripheral blood mononuclear cells (PBMC) cultured with (autologous) tumor cells. T cell responses against tumor cells are enhanced by cytokine transfection of the tumor cells, both in animal models and in *in vitro* human culture systems. (van Elsas et al., 1997; Gansbacher et al., 1990; Tepper et al., 1989; Fearon et al., 1990; Dranoff et al., 1993)

The antigens recognized by the tumor-specific T cells become better defined by the development of molecular cloning techniques. These T cell targets can be divided in three groups: 1) tumor-specific antigens, not expressed in healthy tissues, except testis and placenta (e.g., MAGE, BAGE, GAGE), 2) antigens that are lineage-specific and expressed in both melanoma and melanocytes (e.g., MART-1/ Melan-A, gp100, tyrosinase) and 3) unique, mutated antigens (e.g., β-catenin, CDK4, MUM-1) (reviewed by Van den Eynde and Brichard, 1995).

It was an object of the present invention to identify novel tumor-associated antigens.

To solve the problem underlying the invention, melanoma cell line 518A2 and its IL-2- or GM-CSF-transfectants were compared for their CTL stimulating capacity *in vitro*. Stimulation of autologous PBMC with the IL-2 producing melanoma cells resulted in a melanoma-specific CTL response (van Elsas et al., 1997). CTL clones derived from this culture recognized, besides autologous melanoma cell lines, also a panel of HLA-A*0201 positive melanoma cell lines, but were not reactive with normal melanocytes. Although 518A2 was shown to express a number of antigens previously identified to be recognized by anti-melanoma CTL (van Elsas et al., 1996), the CTL clones available recognize a new melanoma-specific antigen that is immunodominant in 518A2.

In the experiments of the present invention, the target structure that was recognized by one of these CTL clones was fully characterized and named CAMEL (CTL-recognized Antigen on Melanoma). These sequences are described in the attached sequence listing as SEQ ID NO: 1 and SEQ ID NO: 2. Although the identified CAMEL DNA sequence has high homology and is partially identical to NY-ESO-1, a gene originally identified by SEREX technology (Chen et al., 1997, SEQ ID NO: 7), it was surprisingly found that the CTL epitope of CAMEL is encoded by a reading frame (ORF-1) distinct from that encoding the putative LAGE-1 protein (SEQ ID NO: 4) or NY-ESO-1 protein (SEQ ID NO: 8). LAGE-1 is a gene that has recently been identified by Lethé et al., 1998.

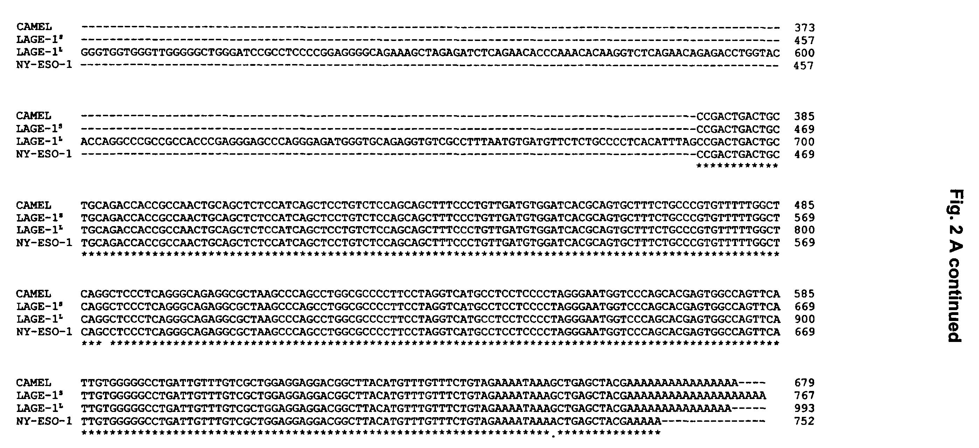

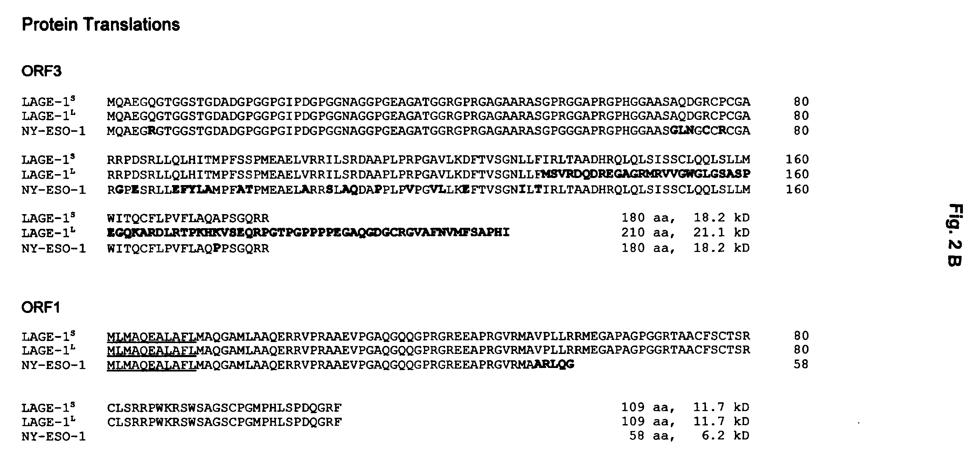

In the present invention, a cDNA clone was identified that lacks the first 86 bp of the LAGE-1^{L} sequence (SEQ ID NO: 5) which means that it is devoid of the initiation codon at position 54 of that sequence (Fig. 2a). The first possible translation initiation site in this clone (4H8) is the ATG at position 94 of LAGE-1^{S} (SEQ ID NO: 3), which is however, not in frame with the first ATG at position 54. Therefore, the CAMEL protein (SEQ ID NO: 2) translated from the 4H8 cDNA clone is different from the putative LAGE-1^{S} protein (SEQ ID NO: 4).

In a first aspect, the present invention is directed to tumor-associated antigens encoded by the ORF-1 of LAGE-1 cDNA and by the ORF-1 of cDNAs hybridizing with LAGE-1.

In the present invention 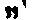 ORF-1" is defined as the open reading frame starting with ATG at position 94 of SEQ ID NO:3 (LAGE-1^{S}), which corresponds to position 10 in SEQ ID NO: 1 (CAMEL), to position 96 in SEQ ID NO: 5 (LAGE-1^{L}) and to position 94 of SEQ ID NO: 7 (NY-ESO-1).

In an embodiment of the invention, the antigen is CAMEL (SEQ ID NO: 2), which is encoded by the ORF-1 of the LAGE-1 cDNA.

In another embodiment, the invention is directed to a polypeptide encoded by the ORF-1 of the NY-ESO-1 cDNA (SEQ ID NO: 7).

Additional members of a gene family including LAGE-1 and NY-ESO1 can be identified by screening cDNA or genomic DNA libraries from cell lines, e.g. cell lines derived from tumors, or from primary tissues, e.g. tumors, testis, placenta, etc. with a probe comprising the ORF-1 of LAGE-1 or NY-ESO-1, at low stringency conditions, and confirming the existence of an open reading frame corresponding to ORF-1 of LAGE-1. An example for low stringency conditions is hybridization at 60°C and washing at 2XSSC at 60°C, or equivalent conditions in Church buffer or SSSP, as described in standard protocols, e.g. Sambrook et al., 1989.

An alternative method that may be used to identify LAGE-1 family members with ORF-1, is Representational Difference Analysis. This PCR-based method has been proven useful to identify genes with tissue-specific or tumor-specific expression (Lethe et al., 1998). By means of this method, LAGE-1 and NY-ESO1 were identified by screening cDNA libraries from melanoma cell lines with a primer from a cDNA clone enriched in melanoma-specific sequences.

In a further aspect, the present invention relates to immunogenic (poly)peptides derived from the tumor-associated antigens of the invention. A first group of peptides is selected from peptides inducing a humoral immune response (induction of antibodies). Such peptides are selected by randomly choosing continuous stretches of amino acids (at least 12-15), applying them to an individual and confirming the generation of antibodies by standard immunological assays, e.g. ELISA. This group of immunogenic (poly)peptides also encompasses the entire antigen or larger fragments thereof.

The second group of peptides, which is preferred, can be presented by MHC molecules (in humans: HLA molecules), they have the potential to induce an immune response, in particular by eliciting a CTL response.

In a preferred embodiment, the immunogenic peptides are derived from CAMEL.

In a preferred embodiment, immunogenic peptides which have the ability to elicit a CTL response, are selected from peptides with the sequence Met Leu Met Ala Gln Glu Ala Leu Ala Phe Leu (SEQ ID NO: 11) or Leu Met Ala Gln Glu Ala Leu Ala Phe Leu (SEQ ID NO: 12)

To obtain peptides that have the ability to elicit a cellular immune response, the selection of peptide sequences from a given antigen is, in the first place, based on the requirement for such peptide to bind to an MHC molecule present in the repertoire of the patient to be treated. Two classes of MHC molecules are distinguished, class I and class II. Class I molecules consist of a membrane-inserted heavy chain and a non-covalently attached light chain. In their structure, MHC class I molecules resemble a moose's head, the antlers forming a groove which is recognized by the peptide. In humans, HLA-A, B and C are the "classical" MHC class I molecules.

Additional immunogenic peptides may be identified by methods known in the art which rely on the correlation between MHC-binding and CTL induction, e.g. those used by Stauss et al., 1992, who identified candidate T-cell epitopes in human papilloma virus.

Since immunogenic peptides can be predicted based on their "peptide binding motif" synthetic peptides which represent CTL epitopes may be designed and synthesized. Several methods, which are useful in the present invention for designing peptides, have been used to identify CTL epitopes from known protein antigens.

It is well established that every MHC class I allelic product has allele-specific requirements for the peptide ligand that binds to its groove and that it ultimately presents. These requirements were summarized as a motif by Falk et al., 1991. A large number of MHC peptide motifs and MHC ligands have become known to date. A method to search a known protein sequence for epitopes fining to a given class I molecule, which is based on this knowledge and which may be used in the present invention, was reviewed by Rammensee et al., 1995. It comprises the following steps: first, the protein sequence is screened for stretches fitting to the basic anchor motif (two anchors in most cases), whereby allowance should be made for some variation in peptide lengths as well as in anchor occupancy. If a motif, for example calls for 9mers with Ile or Leu at the end, 10mers with a fitting C-terminus should be considered as well, and other aliphatic residues at the C-terminus, like Val or Met, should also be considered. In this way, a list of peptide candidates is obtained. These are inspected for having as many non-anchor residues as possible in common with ligand already known, or with the residues listed among the "preferred residues" or "others" on top of each motif (Table, given by Rammensee et al., 1995), for various HLA molecules. Binding assays can be performed at this stage to exclude weak binders which occur frequently among peptides conforming to a basic motif. If a detailed study on peptide binding requirements is available, the candidates can also be screened for non-anchor residues detrimental or optimal for binding (Ruppert et al., 1993). One should keep in mind, however, that pure peptide binding motifs can be misleading in the search for natural ligands, since other constraints, such as enzyme specificity during antigen processing and specificity of transporters or chaperones, are likely to contribute to ligand identity in addition to the MHC binding specificity.

This approach was successfully applied by, inter alia, Kawakami et al., 1995, to identify gp100 epitopes based on known HLA-A2.1 motifs. The validity of the method was confirmed by identifying, in parallel, the epitope regions by using COS cells transfected with cDNA fragments generated by sequential deletion and testing for T-cell reactivity, as described above.

Recently, data bases and prediction algorithms have become available that enable to predict, with great reliability, peptide epitopes that bind to HLA molecules of interest.

Examples for peptide candidates with potential immunogenicity that can be derived from the tumor-associated antigens of the present invention, are the CAMEL-derived peptides with the sequence HLSPDQGRF and LMAQEALAF for HLA-A3 or RMAVPLLRR for HLA-A3101. Similarly, other peptides for these or for other alleles can be determined by the method mentioned above.

The peptide binding can be tested in peptide binding assays. In order to determine the immunogenicity of the selected peptide or peptide equivalent, as defined below, which is the crucial parameter for peptide-based vaccine development and which in most cases strongly correlates with the stability of the peptide-MHC interaction (van der Burg et al, 1996), the methods described by Sette et al., 1994, in combination with quantitative HLA-binding assays, may be used. Alternatively, immunogenicity of the selected peptide may be checked by performing *in vitro* CTL induction by known methods e.g. as described below for *ex vivo* CTL induction.

Alternatively to peptides derived from the naturally expressed tumor antigens, functional equivalents thereof, i.e. peptides with partially altered sequences or substances mimicking peptides, e.g. "peptidomimetics" or retro-inverso peptides, may be obtained by the following methods:

To enhance the immunogenicity of the peptides, amino acid substitutions may be introduced at anchor positions to increase peptide MHC class I-binding affinity. The modified peptides are subsequently evaluated for enhanced binding and immunogenicity by screening for recognition by TIL (tumor-infiltrating lymphocytes) and CTL induction as described by Parkhurst at al, 1996, and Bakker et al., 1997.

Another method useful in the present invention to find more immunogenic peptides by screening peptide libraries with a known CTL was described by Blake et al. 1996; it suggests the use of combinatorial peptide libraries for constructing functional mimics of tumor epitopes recognized by MHC class I-restricted CTLs.

In principle, the selection of peptides capable of eliciting a cellular immune response is carried out in several steps, as described in WO 97/30721, which disclosure is incorporated herein by reference. In short, the candidates are first tested for their binding ability to an MHC molecule; subsequently good binders are tested for immunogenicity. A general strategy for obtaining efficient immunogenic peptides has been described by Schweighoffer, 1997.

The polypeptides of the present invention or immunogenic peptides derived from their sequence, respectively, can be produced recombinantly or by peptide synthesis, as described in WO 96/10413, the disclosure of which is incorporated herein by reference. For recombinant production, a DNA molecule encoding the antigen or the CTL peptide of interest is inserted into an expression vector, transformed or transfected into an appropriate host cell, cultivated under conditions suitable for expression, recovered and purified. For chemical synthesis, various conventional techniques may be used, e.g. commercially available automatic synthesizers.

The tumor antigens of the present invention and the immunogenic peptides derived therefrom or the respective peptide equivalents are useful in cancer therapy, e.g. to induce, in the context of the appropriate MHC presenting molecule, an immunological response to tumors which express the corresponding antigen determinants. The induction of CTLs can be accomplished *in vivo* or *ex vivo*.

For *in vivo* induction of CTLs, a pharmaceutical composition comprising the peptide/antigen is administered to an individual suffering from a tumor associated with the respective tumor antigen in an amount sufficient to elicit an effective CTL response to the antigen-bearing tumor. Thus, the present invention provides pharmaceutical compositions for therapeutic treatment which are intended for parenteral, topical, oral or local administration. Preferably, the compositions are for parenteral administration, e.g. for intravenous, subcutaneous, intradermal or intramuscular application. The peptides/antigens are dissolved or suspended in a pharmaceutically acceptable carrier, preferably an aqueous carrier. The composition may contain additional auxiliary substances, e.g. buffering agents, etc. The peptides may be used alone or in combination with adjuvants, e.g. saponins, alumn, or, in a particularly preferred embodiment, polycations, like polyarginine or polylysine. The peptides may also be linked to components assisting CTL priming, e.g. T helper peptides, lipids or liposomes or coadministered with such components or with immunostimulating substances, e.g. cytokines (IL-2, IFN-γ). Methods and compositions for preparing and administering pharmaceutical compositions for therapeutic treatments are described in WO 95/04542 and WO 97/30721 the disclosures of which are herein incorporated by reference.

The immunogenic peptides may also be used to elicit a CTL response *ex vivo*. An *ex vivo* CTL response to a tumor expressing the antigen is induced by incubating a patient's CTL precursor cells together with antigen presenting cells and the immunogenic peptide. The thus activated CTLs are allowed to mature and expand to effector CTLs which are then readministered to the patient. Alternatively, the tumor antigen may be pulsed onto APCs which present MHC class II-reactive peptides (Mayordomo et al., 1995; Zitvogel et al., 1996). A suitable method for loading peptides onto cells, e.g. dendritic cells, is disclosed in WO 97/19169.

The peptides of the invention are preferably applied as a combination of peptides, e.g. different peptides from one or more antigens of the present invention. In an even more preferred embodiment, the peptides of the invention are combined with peptides derived from other tumor antigens. The selection of the peptides is optimized towards covering multiple HLA types in order to be useful for a broad population of patients and/or towards a broad variety of malignancies, which is taken into account by combining peptides from a large variety of tumor antigens. The number of peptides suitable to be combined to yield an efficient therapy may vary within a broad range, e.g. from about 2 to approximately 100.

In a further aspect, the present invention is directed to isolated DNA molecules comprising ORF-1 of LAGE-1 cDNA and the ORF-1 of cDNAs hybridizing with LAGE-1 under low stringency conditions.

In a further aspect, the invention relates to an isolated cDNA molecule encoding CAMEL.

In a preferred embodiment, the DNA molecule encoding CAMEL comprises nucleotides 54 - 336 of the sequence set forth in SEQ ID NO: 1.

In a further aspect, the invention relates to an isolated DNA molecule comprising ORF-1 of the NY-ESO-1 cDNA (the sequence of NY-ESO-1 is depicted in SEQ ID NO: 9 and 10 for cDNA and protein respectively).

The DNAs of the present invention, or the corresponding RNAs, may be used, as an alternative to the use of the protein or the peptide, for cancer immunotherapy. Alternatively to using the natural sequence or fragments thereof, engineered derivatives may be utilized. These include sequences modified to encode (poly)peptides with improved immunogenicity, e.g. taking into account the modifications described above for the peptides. Another form of modification is the assembly of multiple sequences encoding immunologically relevant peptides in a so-called string-of-beads fashion, as described by Toes et al., 1997. The sequences may also modified by adding auxiliary coding elements, e.g. targeting functions that ensure more efficient delivery and processing of the immunogen (e.g. Wu et al., 1995).

The nucleic acid molecules may be delivered either directly or as part of a recombinant virus or bacterium. Recombinant In principle, any method that is known for gene therapy may be applied for nucleic acid-based cancer immunotherapy, both *in vivo* and *ex vivo*.

Examples for *in vivo* delivery are direct injection (injection of "naked" DNA) either intramuscularly or by "gene gun", which has been shown to result in the generation of CTLs against tumor antigens. Examples for recombinant organisms are vaccinia virus, fowlpox virus and adenovirus or Listeria monocytogenes (see Coulie, 1997 for a comprehensive review). Furthermore, synthetic nucleic acid carriers like cationic lipids, microspheres, microbeads, liposomes may be useful for *in vivo* delivery of the sequence encoding respective antigen/peptide. Similarly as for peptides, various auxiliary agents that enhance the immune response may be co-applied, e.g. cytokines, either as proteins or as plasmids encoding these.

Examples for *ex vivo* delivery are transfection of dendritic cells (Tuting, T., 1997) or other antigen presenting cells which are applied as a cellular cancer vaccine.

The present invention is also directed to the use of cells that express the tumor-associated antigens of the invention, either naturally or upon transfection with the respective coding sequence, for the preparation of a tumor vaccine.

In the present invention, it has been shown that CTL clones raised against IL-2 producing melanoma cell line 518/IL-2.14 are reactive against two alternatively spliced variants of LAGE-1, LAGE-1^{S} (SEQ ID NO: 3) and LAGE-1^{L} (SEQ ID NO: 5) and NY-ESO-1 (SEQ ID NO: 9). NY-ESO-1 is a recently described tumor antigen, identified by screening a cDNA library of an esophagus carcinoma with autologous patient serum (SEREX-method (Chen et al., 1997)). NY-ESO-1 is expressed in different tumor types but not in healthy tissues except the testis.

In the present invention, the epitope of specific CTL 1/29 was determined by cDNA expression cloning and a truncated LAGE-1 cDNA clone was found. This truncation led to the identification of the peptide epitope in an alternative reading frame, since the "normal" translation initiation site of LAGE-1 was absent. However, COS/HLA-A*0201 cells transfected with full length LAGE-1 or NY-ESO-1 cDNA clones could stimulate the CTL clone to TNF production as well. This probably means that two different proteins can be translated from one single mRNA.

NY-ESO-1 also has been described as the target of melanoma-specific HLA-A*0201 restricted CTL clones, which recognize a an epitope translated in ORF3, located between aa 155 and 167 (Jager et al., 1998). Therefore, it is very likely that also LAGE-1^{S} will be recognized by these clones, but not LAGE-1^{L}, since the protein sequence is different at this part of the molecule. Our CTL clones recognize a peptide in an alternative reading frame, which is encoded in both LAGE-1 and NY-ESO-1. This means that tumor cells expressing either LAGE-1 or NY-ESO-1 can be recognized by MLMAQEALAFL-specific CTL, which might enlarge the number of tumors that can be treated with immunotherapy based on this peptide.

### Brief description of the Figures:

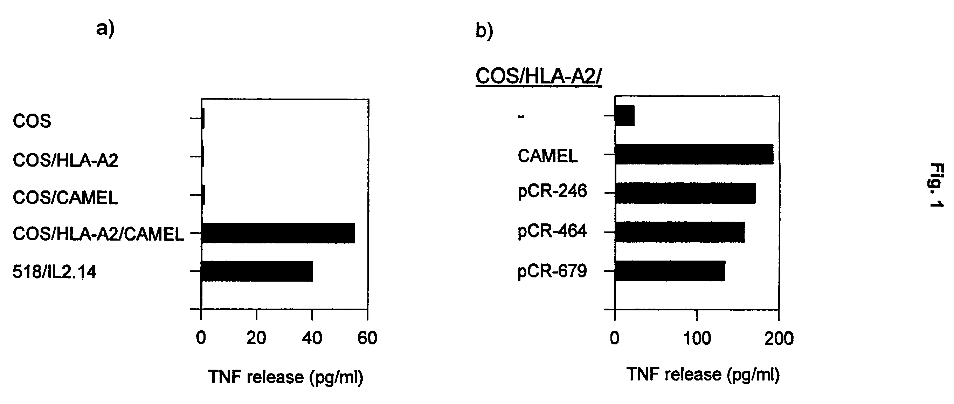

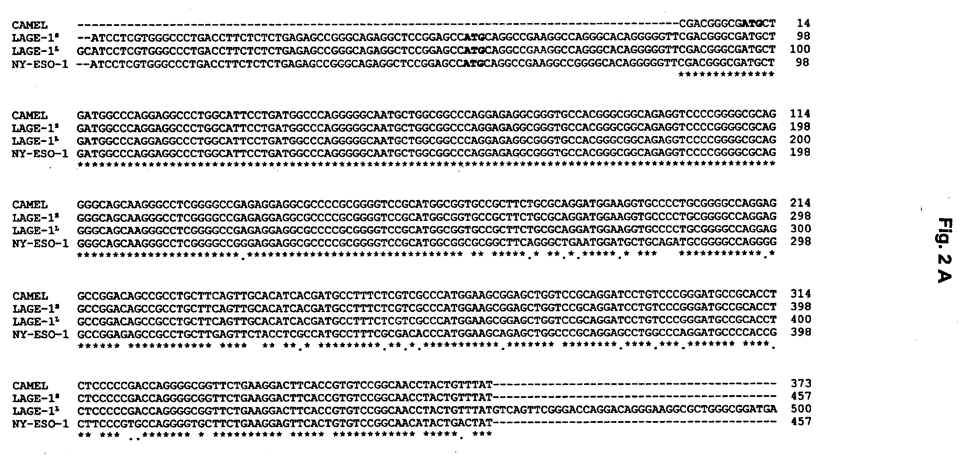

- **Figure 1**:: COS-7 transfection experiments with cDNA clone CAMEL and deletion constructs
a) COS-7 cells were transfected with cDNAs as indicated and tested with CTL 1/29 in a TNF release assay.
b) Deletion constructs of CAMEL cDNA were cotransfected with HLA-A*0201 cDNA in COS-7, followed by a TNF release assay with CTL 1/29. The PCR clones contain the numbers of nucleotides of the CAMEL cDNA as indicated.
- **Figure 2**:: a) Nucleotide alignment of cDNA clones CAMEL, LAGE-1^{S}, LAGE-1^{L} and NY-ESO-1.
b) Protein translations of the cDNA clones LAGE-1^{S}, LAGE-1^{L} and NY-ESO-1. The translation of CAMEL is identical to the translation of LAGE-1^{S/L} in ORF1. Although ORF3 seems the most putative one, the CTL epitope is encoded in ORF1 (underlined).
- **Figure 3**:: Characterisation of peptides recognized by CTL clone 1/29
a) TNF release assay with predicted HLA-A*0201 binding CAMEL peptides. Peptides as indicated were loaded on BLM, an HLA-A*0201* melanoma cell line, at a concentration of 10 µg/ml and tested with CTL 1/29 in a TNF release assay.
b) The effects of increasing concentrations of peptides, derived from the major target epitope MLMAQEALAFL on recognition by CTL 1/29. Various concentrations of peptides as indicated were loaded on HLA-A*0201⁺ cells and tested in a TNF release assay with CTL 1/29.

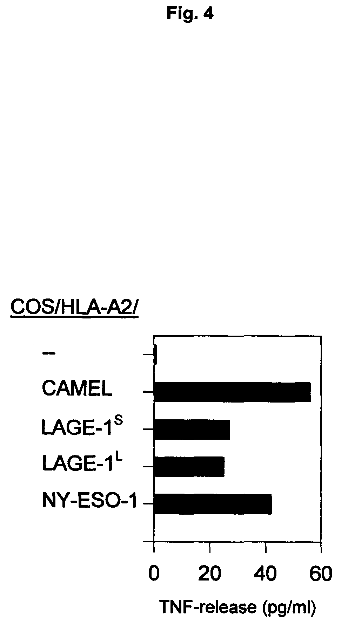

- **Figure 4**:: LAGE-1^{S/L} (and NY-ESO-1) both encode the CTL epitope
COS/HLA-A*0201 cells were transfected with these cDNA clones and reactivity with CTL 1/29 was measured in a TNF release assay.

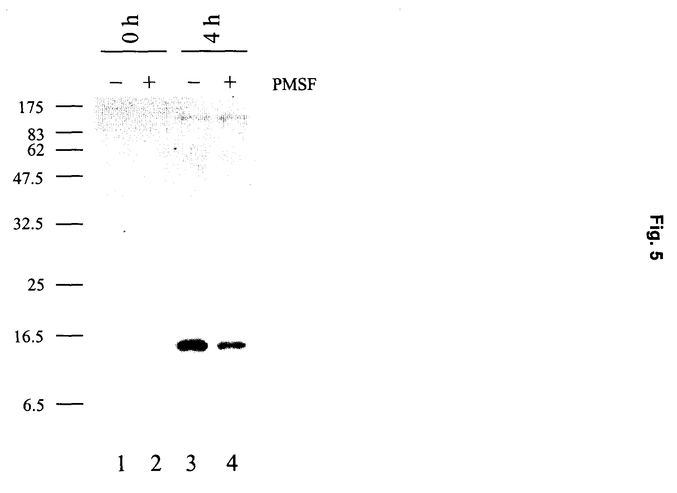

- **Figure 5**:: His-tagged CAMEL protein, synthesized in E.coli

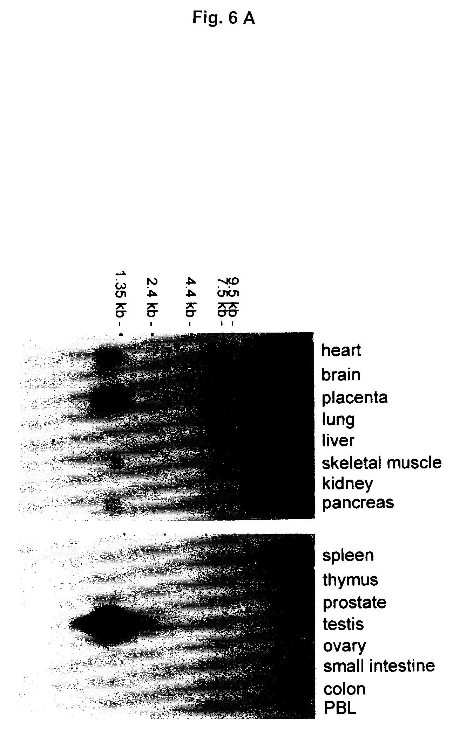

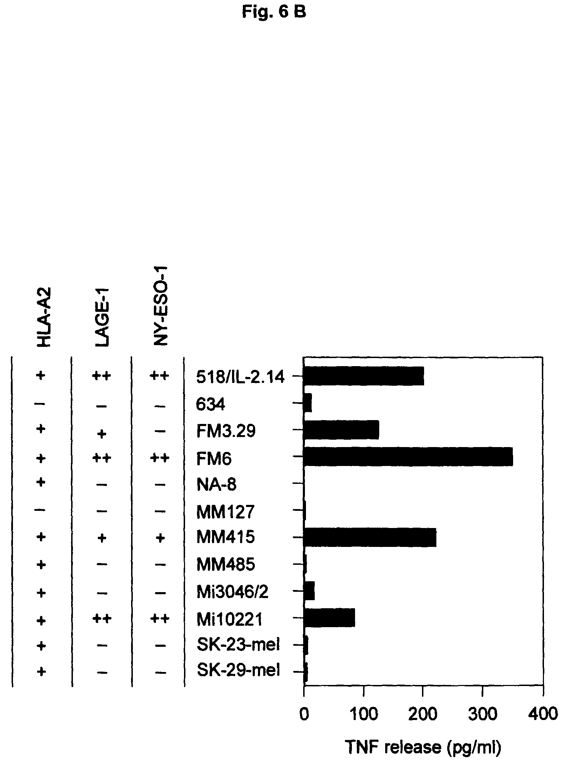

- **Figure 6**:: Expression of LAGE-1^{S/L} and NY-ESO-1 in healthy human tissues and melanoma cell lines
a) Northern Blot analysis of the expression of LAGE-1/NY-ESO-1 in a panel of healthy human tissues as indicated. The Blot was hybridised with ³²P-dCTP-labeled LAGE-1^{S} cDNA.
b) RT-PCR for LAGE-1 and NY-ESO-1. To discriminate between LAGE-1 and NY-ESO-1 mRNA, the same panel of melanoma cell lines was analysed by RT-PCR with gene-specific primers. Melanoma cell lines as indicated were used as targets in a TNF release assay with CTL 1/29.

### Brief description of the sequences:

SEQ ID NO: 1: CAMEL (H8) cDNA sequence and translation
SEQ ID NO: 2: CAMEL protein sequence
SEQ ID NO: 3: LACE-1^{S} cDNA sequence and translation
SEQ ID NO: 4: LAGE-1^{S} protein sequence
SEQ ID NO: 5: LAGE-1^{L} cDNA sequence and translation
SEQ ID NO: 6: LAGE-1^{L} protein sequence
SEQ ID NO: 7: NY-ESO-1 cDNA sequence and translation
SEQ ID NO: 8: NY-ESO-1 protein sequence
SEQ ID NO: 9: NY-ESO-1 cDNA and alternative translation
SEQ ID NO: 10: protein sequence of alternatively translated NY-ESO-1
SEQ ID NO: 11: peptide sequence of the CAMEL CTL epitope (11-mer)
SEQ ID NO: 12: peptide sequence of the CAMEL CTL epitope (10-mer)
SEQ ID NO: 13: oligonucleotide SP6F-pSV
SEQ ID NO: 14: oligonucleotide R1
SEQ ID NO: 15: oligonucleotide R2
SEQ ID NO: 16: oligonucleotide T7R-pSV
SEQ ID NO: 17: oligonucleotide F3
SEQ ID NO: 18: oligonucleotide ESO-1B
SEQ ID NO: 19: oligonucleotide ESO-1A
SEQ ID NO: 20: oligonucleotide 4H8-A
SEQ ID NO: 21: oligonucleotide 4H8-C

In the Examples, if not stated otherwise, the following materials and methods were used

### a) Cell cultures

Melanoma cell lines and COS-7 cells were maintained in DMEM containing 4.5 mM glucose supplemented with 8% FCS, 2 mM L-glutamine, 100 µg/ml of each penicillin and streptomycin. Melanoma cell line 518A2 was established in our laboratory from the dissected metastasis of a male patient in 1985, as described before (Versteeg et al., 1988). An IL-2 producing variant, 518/IL-2.14, was obtained by transfection of 518A2 with the IL-2 cDNA (Osanto et al., 1993). Other melanoma cells that were used as targets in TNF release assay are FM3.29, FM6, FM28.4 and FM55P (gifts from J. Zeuthen, Denmark), MM127, MM415, MM485 (gifts from N. Hayward, Australia), SK-MEL-23, SK-MEL-29 (obtained from T. Wölfel, Mainz), Mi10221, Mi3046/2, NA8, BLM (obtained from M. Visseren, Leiden). EBV-transformed B-LCL and the TNF-sensitive WEHI-164 clone 13 (a gift from Dr. P. Coulie, Brussels) were cultured in RPMI-1640, supplemented with L-glutamine and antibiotics as above, and 10% FCS.

With the IL-2 producing cell line 518/IL-2.14 and autologous peripheral blood mononuclear cells (PBMC) a CTL induction was performed, resulting in melanoma-specific HLA-A*0201 restricted CTL clones (van Elsas et al., 1997). The identification of the epitope of one of these clones, CTL 1/29, is reported here.

### b) cDNA expression cloning

A cDNA library of 518/IL-2.14 was constructed in the expression vector pSVsport1 (GIBCO, BRL) using the Superscript Plasmid System (GIBCO, BRL). As to that purpose, poly-A⁺ mRNA was isolated using the Fast-Track system (Invitrogen), followed by reverse-transcription with an oligo-dT/NotI primer. SalI adapters were ligated to ds-cDNA and after NotI digestion and size fractionation, cDNA fragments were cloned into the pSVsport1 vector digested with SalI and NotI. After electroporation into ElectroMAX-DH10B (GIBCO, BRL) (following the manufacturers instructions) and selection for ampicilin resistance, 50-100 colonies were pooled for mini DNA isolation (QIAprep 8 plasmid kit, Qiagen). The in this way obtained cDNA pools were transfected in duplicate into COS-7 cells, together with the restriction element HLA-A*0201 (pBJ1.neo/HLA-A*0201, (Lin et al., 1990)), using the DEAE-dextran method. Briefly, COS-7 cells were seeded in 96-wells flatbottom plates at 1.5x10⁴ cells per well in 100 µl DMEM, 8% FCS. After 2 hours, medium was replaced by 30 µl transfection mixture, containing 100 ng cDNA pool, 100 ng HLA-A*0201 cDNA, 400 ng/ml DEAE-dextran and 100 µM chloroquine in serum free DMEM. Cells were incubated for 4 hours at 37°C and shocked for 2 minutes by the addition of 50 µl 10% DMSO in PBS. The shock medium was replaced by 200 µl DMEM, 8% FCS, and 48 hours later the cells were used as target cells for CTL in a TNF release assay.

### c) Deletion constructs

Deletion constructs of cDNA clone 4H8 were obtained by PCR. PCR products were cloned in vector pCR3.uni (TA cloning system, Invitrogen). The constructs pCR-246 and pCR-464 were made with the vector-based forward primer, SP6F-pSV (SEQ ID NO: 13) and the reverse primers in cDNA 4H8, R1 (SEQ ID NO: 14) and R2 (SEQ ID NO: 15) respectively. As a control the complete 679 bp cDNA was cloned by PCR with two primers on the pSVsport vector, SP6F-pSV (SEQ ID NO: 13) and T7R-pSV (SEQ ID NO: 16), resulting in pCR-679.

### d) TNF release assay

CTL reactivity against tumor target cells, transfected COS-7 or peptide loaded cells was measured in a TNF release assay. Target cells were seeded in duplicate or triplicate at 1.5-2x10⁴ cells per well in a 96-wells flat bottom plate and 1500-2000 CTL were added to each well, in a total volume of 100 µl / well (IMDM, supplemented with antibiotics and 5% FCS). After 24 hours of co-culturing of effector and target cells, 50 µl out of each well was added to a fresh 96-wells flatbottom plate, containing 50 µl (4.5x10⁴) TNF-sensitive WEHI-164 cells per well in IMDM, supplemented with antibiotics, 5% FCS, 2 µg/ml Actinomycin D and 40 mM LiCl. A viability staining was performed 24 hours later by the addition of 50 µl of 3-(4,5dimethylthiazol-2-yl)-2,5 diphenyltetrazolium bromide (MTT) solution (2.5 mg/ml in PBS). After incubation for 2-4 hours at 37°C the OD₅₅₀₋₆₅₀ was measured. TNF release in pg/ml was calculated from a standard with known TNF concentrations.

### e) Northern Blot analysis

To determine expression in healthy tissues a Multiple Tissue Northern Blot was obtained commercially (Clontech). As a probe, LAGE-1 cDNA was used, labeled with γ-³²P-dCTP by use of the Mega-Prime Labeling kit (Amersham).

### f) RT-PCR

cDNA synthesis was performed using oligo-dT and M-MLV reverse transcriptase (Promega). Primers used for LAGE-1 specific PCR were the F3 (SEQ ID NO: 17) and ESO-1B primer (SEQ ID NO: 18). ESO-1B was also used as a reverse primer in the NY-ESO-1-specific PCR, while ESO-1A (SEQ ID NO: 19) was the forward primer in this reaction (Chen et al., 1997). Reactions were performed in a Biometra-Uno or -Trio programmed as follows: 5 minutes 95°C, 30 cycles of 1 min. 95°C, 1 min. 58°C, 1 min. 72°C, followed by 10 minutes 72°C.

### g) Expression of CAMEL in E. Coli

A fragment containing the coding sequence of CAMEL was made by PCR with the following primers:
4H8-A: GAAGAACATATGCTGATGGCCCAGGAGGC (SEQ ID NO: 20)
4H8-C: TTAAAGATCTCAGAACCGCCCCTGGTCG (SEQ ID NO: 21)

This fragment was digested with NdeI and BglII and cloned in the NdeI and BamHI sites of vector pET19b (Novagen, Madison, WI). This vector contains a 6xHis-tag coding sequence, allowing detection of the His-tagged protein with an anti-His antibody. The pET19b-CAMEL construct was transformed into BL21(DE3)pLysS E. coli bacteria (Novagen, Madison, WI). After culturing the bacteria at 30°C until an OD₆₀₀ =0.5, IPTG (1 mM) was added to induce overexpression of the His-tagged CAMEL protein. Samples were taken at 0h and 4h after IPTG induction and lysates of these samples were tested on a Western Blot with the Penta-His Antibody (Qiagen) according to the Western and Colony Blot protocol of the supplier. The His-tagged protein was visualized using the SuperSignal Substrate system for Western blotting (Pierce, Rockford, US).

### Example 1

### cDNA clone 4H8 (CAMEL) encodes the target for melanoma-specific CTL1/29

The antigenic epitope of melanoma-specific CTL 1/29 was identified by the expression of cDNA library 518/IL2.14 and the restriction element HLA-A*0201 in COS-7 cells, followed by CTL screening in a TNF release assay. A positive pool of cDNAs was subcloned and clone 4H8, called CAMEL (SEQ ID NO: 1), was found to stimulate TNF release by the CTL to a similar extent as the original 518/IL2.14 cell line (Fig. 1). COS-7 cells or COS-7 cells transfected with HLA-A*0201 or the 4H8 cDNA only were not recognized. The isolated 4H8 cDNA clone has a 679 bp insert, which shows strong homology with NY-ESO-1 (SEQ ID NO: 7), a tumor antigen originally identified as a target for humoral immune responses by serum screening methods (SEREX) (Chen et al., 1997). Colony hybridization of the cDNA library, using clone 4H8 as a probe resulted in the detection of 2 types of full length clones which we call LAGE-1^{S} (SEQ ID NO: 3) and LAGE-1^{L} (SEQ ID NO: 5) (Fig. 2a). LAGE-1^{L} contains a 229 bp insertion at position 457, which has the consensus sequences for an intron, starting with a 5' GT and ending 3' AG. This indicates alternative splicing of LAGE-1 mRNA. However, cDNA clone 4H8 lacks the first 84 bp of the LAGE-1 cDNA sequence.

### Example 2

### The peptide epitope of CTL 1/29 is coded in an alternative reading frame of LAGE-1 or NY-ESO-1

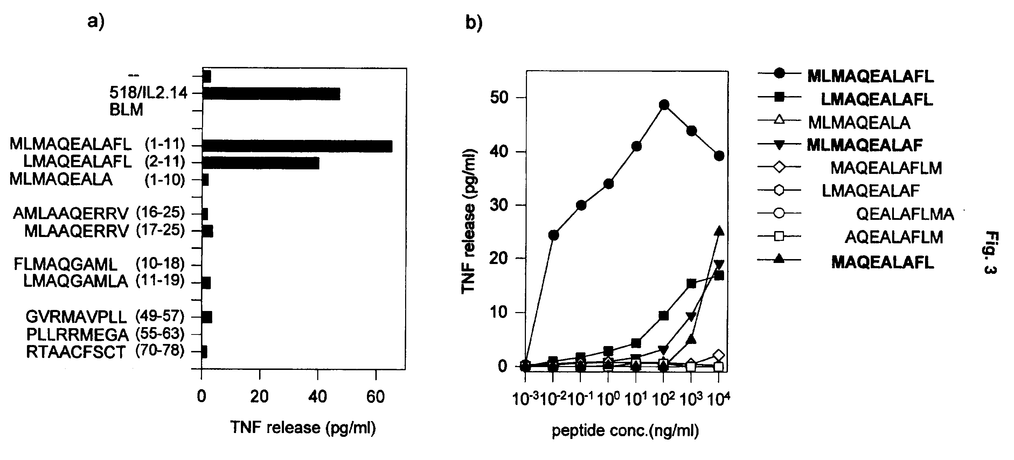

To identify which peptide was recognized by CTL 1/29, deletion constructs of cDNA 4H8 were transfected in HLA-A*0201⁺ COS-7 cells and tested in a TNF release assay. CTL reactivity was measured with all constructs (Fig. 1b), indicating that the epitope was coded within the first 330 bp of clone 4H8. An HLA-A*0201 binding motif search was performed on the predicted protein sequence of that region (Drijfhout et al., 1995; D'Amaro et al., 1995), presuming that the ATG at position 10 in 4H8 functions as the translation initiation site. Predicted strong binding peptides at regions 1-11, 2-11, 1-9, 10-18, 11-19, 16-25, 17-25, 49-57, 55-63 and 70-78 of the CAMEL protein sequence (Fig. 2b) were added to HLA-A*0201* BLM melanoma cells, and tested for CTL reactivity in a TNF release assay (Fig. 3b).

At a peptide concentration of 10 µg/ml only the N-terminal 11- and 10-mer peptides (M)LMAQEALAFL (SEQ ID NO: 11 and NO: 12) induced preponderant recognition by CTL 1/29 (Fig. 3a), indicating that the epitope recognized by the CTL is located in the first 11 amino acids of the CAMEL-encoded protein. Closer inspection of peptides derived of this N-terminal 11-mer in a peptide concentration dependent TNF release assay (Fig. 3b) revealed that the methionine at position 1 as well as the leucine at position 11 are of crucial importance for reconstituting CTL reactivity. Deletion of either of these amino acids leads to an at least 5 decades higher peptide concentration required for comparable TNF release. The only other peptide showing weak activity is the 3-11 MAQEALAFL. In contrast, the MLMAQEALA has no activity at all (Fig. 3b), suggesting that the C-terminal amino acids FL do significantly contribute to the recognition by the CTL.

### Example 3

### Comparison of CAMEL, LAGE-1^{S/L}, NY-ESO-1

As already mentioned, cDNA clone 4H8 lacks the first 84 bp of the LAGE-1 sequence, which means that it is devoid of the initiation codon at position 54 of that sequence (Fig. 2a). The first possible translation initiation site in 4H8 corresponds with the ATG at position 94 of LAGE-1, which is however, not in frame with the first ATG at position 54. Therefore, the protein translated from the 4H8 cDNA clone is different from the putative LAGE-1 protein, since translation takes place in another reading frame (Fig. 2a and b). 4H8 encodes a protein of 109 amino acids (SEQ ID NO: 2) with a predicted molecular weight of 11.7 kD. The LAGE-1^{S} protein translated from the first ATG will be a 180 aa protein of 18.2 kD (SEQ ID NO: 4), while the unspliced variant, LAGE-1^{L}, encodes a 210 aa protein of 21.1 kD (SEQ ID NO: 6). NY-ESO-1 protein (SEQ ID NO: 8) is probably of the same size as LAGE-1^{S}, but differs at 26 amino acids. However, if translation of LAGE-1^{S/L} starts at the second ATG, proteins will be translated in another reading frame and are in that case identical to the protein translated from cDNA 4H8. Alternative translation of NY-ESO-1 (SEQ ID NO: 9 and NO: 10) results in a shorter variant of this protein (58 amino acids), because of an earlier stop codon (Fig. 2b), which differs from the CAMEL protein sequence only in its last 5 amino acids (Fig. 2b).

It was examined whether cells transfected with the complete LAGE-1 (or NY-ESO-1) cDNA clones are able to stimulate CTL 1/29. Remarkably, COS/HLA-A*0201 cells transfected with LAGE-1^{S}, the alternatively spliced LAGE-1^{L}, (as well as with the NY-ESO-1) cDNA are able to stimulate CTL 1/29 (Fig. 4). This indicates that, at least in COS-7 cells, protein translation also starts from the second start codon at nucleotide 94 in LAGE-1^{S}, notwithstanding the presence of the first ATG at position 54. Also in this case, this results in the "alternative reading frame" peptide, MLMAQEALAFL, recognized by CTL 1/29.

### Example 4

### Expression of CAMEL in E. Coli

To investigate whether CAMEL is indeed translated from the ORF-1 of the CAMEL (4H8) cDNA, the CAMEL cDNA (SEQ ID No: 1) was cloned in a bacterial expression vector (pET19b) (Studier et al., 1990). This vector contains a 6xHis-tag coding sequence, allowing detection of the His-tagged protein with an anti-His antibody. The pET19b-CAMEL construct was transformed into E.coli and the bacteria were treated with IPTG to induce expression of the His-tagged CAMEL protein. Extracts were analyzed by Western blotting using the Penta-His antibody. Western blotting of a lysate shows a 15.5 kD protein, only slightly higher than the expected 14.5 kD of the His-tagged CAMEL protein after staining with a anti-His antibody (Fig. 5).

The CAMEL cDNA (SEQ ID No: 1) was cloned in pET19b and expressed in E.Coli. Lanes 1 and 2 represent the samples taken at 0h, lanes 3 and 4 at 4h after induction with IPTG. Because CAMEL might be an unstable protein, induction of protein expression was performed in the absence (lanes 1 and 3) or presence (lanes 2 and 4) of PMSF (a protease inhibitor). At the left the positions of the molecular weight marker proteins are indicated.

### Example 5

### Expression of LAGE-1 and NY-ESO-1 in healthy human tissues and melanoma cell lines

Hybridisation of Multiple Tissue Northern blots containing RNA of healthy human tissues with the LAGE-1^{S} cDNA showed high expression in testis and placenta and low, (but clear) expression in heart, skeletal muscle and pancreas (Fig. 6a). The positive signals exist of two bands, probably reflecting LAGE-1^{S}/NY-ESO-1 (750 bp) and LAGE-1^{L} (1000 bp).

Several melanoma cell lines were tested for expression of LAGE-1 and NY-ESO-1 by(Northern Blot analysis and) RT-PCR (Fig. 6b). Because of the strong homology between both genes, it is not possible to discriminate between LAGE-1 and NY-ESO-1 on Northern Blot. Therefore RT-PCR was performed with specific primers. We found in most cell lines a correlated expression of LAGE-1 and NY-ESO-1; only cell line FM3.29 had expression of LAGE-1, but was negative for NY-ESO-1. Other cell lines expressed either both or none of the two genes (Fig. 6b). There was a good correlation between the level of expression and the recognition by CTL 1/29 (Fig. 6b).

### Bibliography

Bakker, A.B.H., van der Burg, S.H., Huijbens, R.J.F., Drijfhout, J.-W., Melief, J.M., Adema, G.J., and Figdor, C.G. (1997). Analogues of CTL epitopes with improved MHC Class-I Binding capacity elicit anti-melanome CTL recognizing the wild-type epitope. Int. J. Cancer 70, 302-309.

Blake, J., Johnston, J.V., Hellström, K.E., Marquardt, H., and, Chen, L. (1996). Use of Combinatorial Peptide Libraries to Construct FunctionalMimics of Tumor Epitopes Recognized by MHC Class I-Restricted Cytolytic T Lymphocytes. J. Exp. Med. 184, 121-130.

Chen, Y.T., Scanlan, M.J., Sahin, U., Tureci, O., Gure, A.O., Tsang, S., Williamson, B., Stockert, E., Pfreundschuh, M., and Old, L.J. (1997). A testicular antigen aberrantly expressed in human cancers detected by autologous antibody screening. Proc. Natl. Acad. Sci. U. S. A. *94*, 1914-1918.

Coulie P.G. (1997) Human tumour antigens recognized by T cells: new perspectives for anti cancer vaccines. Mol. Med. Today 3, 261-268

D'Amaro, J., Houbiers, J.G., Drijfhout, J.W., Brandt, R.M., Schipper, R., Bavinck, J.N., Melief, C.J., and Kast, W.M. (1995). A computer program for predicting possible cytotoxic T lymphocyte epitopes based on HLA class I peptide-binding motifs. Hum. Immunol. *43*, 13-18.

Dranoff, G., Jaffee, E., Lazenby, A., Golumbek, P., Levitsky, H., Brose, K., Jackson, V., Hamada, H., Pardoll, D., and Mulligan, R. (1993). Vaccination with irradiated tumor cells engineered to secrete murine granulocyte-macrophage colony-stimulating factor stimulates potent, specific, and long-lasting anti-tumor immunity. Proc. Natl. Acad. Sci. USA *90*, 3539-3543.

Drijfhout, J.W., Brandt, R.M., D'Amaro, J., Kast, W.M., and Melief, C.J. (1995). Detailed motifs for peptide binding to HLA-A*0201 derived from large random sets of peptides using a cellular binding assay. Hum. Immunol. *43*, 1-12.

Falk, K., Rötzschke, O., Stevanovi*'*c, S., Jung, G., and Rammensee, H-G (1991). Allele-specific motifs revealed by sequencing of self-peptides eluted from MHC molecules. Nature *351*, 290-296.

Fearon, E.R., Pardoll, D.M., Itaya, T., Golumbek, P., Levitsky, H.I., Simons, J.W., Karasuyama, H., Vogelstein, B., and Frost, P. (1990). Interleukine-2 production by tumor cells bypasses T helper function in the generation of an antitumor response. Cell *60*, 397-403.

Gansbacher, B., Zier, K., Daniels, B., Cronin, K., Bannerji, R., and Gilboa, E. (1990). Interleukin 2 gene transfer into tumor cells abrogates tumorigenicity and induces protective immunity. J. Exp. Med. *172*, 1217-1224.

Jager, E., Chen, Y.T., Drijfhout, J.W., Karbach, J., Ringhoffer, M., Jager, D., Arand, M., Wada, H., Noguchi, Y., Stockert, E., Old, L.J., and Knuth, A. (1998). Simultaneous humoral and cellular immune response against cancer-testis antigen NY-ESO-1: definition of human histocompatibility leukocyte antigen (HLA)-A2-binding peptide epitopes. J. Exp. Med. *187*, 265-270.

Kawakami, Y., Eliyahu, S., Jennings, C., Sakaguchi, K., King, X., Southwood, S., Robbins, P.F., Sette, A., Appella, E., and Rosenberg, S.A. (1995). Recognition of multiple epitopes in the human melanoma antigen gp100 by tumor-infiltrating T lymphocytes associated with *in vivo* tumor regression. J. Immunol. *154*, 3961-3968.

Lethe, B., Lucas, S., Michaux, L., Desmet, C., Godelaine, D., Serrano, A., Deplaen, E. and Boon, T. (1998). LAGE-1, a new gene with tumor specificity. Int. J. Cancer *76* (6):903-908.

Lin, A.Y., Devaux, B., Green, A., Sagerström, C., Elliott, J.F., and Davis, M.M. (1990). Expression of T Cell Antigen Receptor Heterodimers in a Lipid-Linked Form. Science *249*, 677-679.

Mayordomo, J.I., Zorina, T., Storkus, W.J., Zitvogel, L., Celluzzi, C., Falo, L.D., Melief, C.J., Ildstad, S.T., Kast, W.M., DeLeo, A.B., and Lotze, M.T. (1995). Bone marrow-derived dendritic cells pulsed with synthetic tumour peptides elicit protective and therapeutic antitumour immunity. Nature Medicine *1*, 1297-1302.

Osanto, S., Brouwenstyn, N., Vaessen, N., Figdor, C.G., Melief, C.J., and Schrier, P.I. (1993). Immunization with interleukin-2 transfected melanoma cells. A phase I-II study in patients with metastatic melanoma. Hum. Gene Ther. *4*, 323-330.

Parkhurst, M.R., Salgaller, M.L., Southwood, S., Robbins, P.F., Sette, A., Rosenberg, S.A., and Kawakami, Y. (1996). Improved induction of melanoma-reactive CTL with peptides from the melanoma antigen gp100 modified at HLA-A*0201-binding residues. J. Immunol. *157*, 2539-2548.

Rammensee, H.G. and Friede, T. (1995). MHC ligands and peptide motifs: first listing. Immunogenetics *41*, 179-228.

Ruppert, J., Sidney, J., Celis, E., Kubo, R.T., Grey, H.M., and Sette, A. (1993). Prominent role of secondary anchor residues in peptide binding to HLA-A2.1 molecules. Cell *74*, 929-937.

Sambrook, J., Fritsch, E.F., and Maniatis, T. (1989). Molecular Cloning, a Laboratory Manual (New York: Cold Spring Harbor Laboratory Press).

Schweighoffer, T. (1997) Molecular cancer vaccines: tumor therapy using antigen-specific immunizations. Path. Onc. Res. *3*, 164-176

Sette, A., Vitiello, A., Reherman, B., Fowler, P., Nayersina, R., Kast, W.M., Melief, C.J.M., Oseroff, C., Yuan, L., Ruppert, J., Sidney, J., del Guercio, M.-F., Southwood, S., Kubo, R.T., Chesmut, R.W., Grey, H.M., and Chisari, F.V. (1994). The relationship between class I binding affinity and immunogenicity of potentioal cytotoxic T Cell epitopes. J. Immunol. *153*, 5586-5592.

Stauss, H.J., Davies, H., Sadovnikova, E., Chain, B., Horowitz., and Sinclair, C. (1992). Induction of cytotoxic T lymphocytes with peptides *in vitro*: Identification of candidate T-cell epitopes in human papilloma virus. Proc. Natl. Acad. Sci. USA. *89*, 7871-7875.

Studier, F.W., Rosenberg, A.H., Dunn, J.J. and Dubendorf, J.W. (1990). Use of T7 RNA polymerase to direct expression of cloned genes. Methods in Enzymology *185*, 60-89

Tepper, R.I., Pattengale, P.K., and Leder, P. (1989). Murine interleukine-4 displays potent anti-tumour activity *in vivo*. Cell *57*, 503-512.

Toes, R.E., Hoeben, R.C., Van der Voort, E., Ressing, M.E., Van-der-Eb, A.J. Melief C.J.M., and Offringa, R. 1997 Protective anti-tumor immunity induced by vaccination with recombinant adenoviruses encoding multiple tumor-associated cytotoxic T lymphocyte epitopes in a string-of-beads fashion. Proc. Natl. Acad. Sci. U.S.A. *94* (26):14660-14665

Tuting, T., DeLeo, A.B., Lotze, M.T., and Storkus, W.J. Genetically modified bone marrow-derived dendritic cells expressing tumor-associated viral or "self" antigens induce antitumor immunity *in vivo*. Eur. J. Immunol. 27, 2702-2707, 1997.

van der Burg, S.H., et al., (1996), Immunogenicity of peptides bound to MHC class I molecules depends on the MHC-peptide complex stability. J. Immunol. *156*, 3308-3314

Van den Eynde, B. and Brichard, V.G. (1995). New tumor antigens recognized by T cells. Current Opinion in Immunology *7*, 674-681.

van Elsas, A., van der Minne, C.E., Borghi, M., van der Spek, C.W., Braakman, E., Osanto, S., and Schrier, P.I. (1996). CTL Recognition of an IL-2 Producing Melanoma Vaccine. In: Immunology of human melanoma. Tumor-host interaction and immunotherapy, edited by M. Maio, Amsterdam: IOS, 1996, p. 165-173.

van Elsas, A., Aarnoudse, C., van der Minne, C.E., van der Spek, C.W., Brouwenstijn, N., Osanto, S., and Schrier, P.I. (1997). Transfection of IL-2 augments CTL response to human melanoma cells *in vitro*: immunological characterization of a melanoma vaccine. Journal of Immunotherapy *20*, 343-353.

Versteeg, R., Noordermeer, I.A., Krüse-Wolters, K.M., Ruiter, D.J., and Schrier, P.I. (1988). c-Myc downregulates class I HLA expression in human melanomas. EMBO J. *7*, 1023-1029.

Wu, T.C., Guarnieri, F.G., Staveley-O'Carroll, K.F., Viscidi, R.P., Levitsky, H.I., Hedrick, L., Cho, K.R., August, J.T., and Pardoll, D.M. (1995). Engineering an intracellular pathway for major histocompatibility complex class II presentation of antigens. Proc. Natl. Acad. Sci. U.S.A. *92*(25): 11671-11675.

Zitvogel, L., Mayordomo, J.I., Tjandrawan, T., DeLeo, A.B., Clarke, M.R., Lotze, M.T., and Storkus, W.J. (1996). Therapy of murine tumors with tumor peptide-pulsed dendritic cells: dependence on T cells, B7 costimulation, and T helper cell 1-associated cytokines. J. Exp. Med. *183*, 87-97.

SEQUENCE LISTING

(1) GENERAL INFORMATION:

(i) APPLICANT:
(A) NAME: Boehringer Ingelheim International GmbH
(B) STREET: Binger Strasse 173
(C) CITY: Ingelheim am Rhein
(E) COUNTRY: Germany
(F) POSTAL CODE (ZIP): 55216
(G) TELEPHONE: 06132/772282
(H) TELEFAX: 06132/774377

(ii) TITLE OF INVENTION: Tumor-associated antigens

(iii) NUMBER OF SEQUENCES: 21

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 679 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: NO

(iv) ANTI-SENSE: NO

(vi) ORIGINAL SOURCE:
(A) ORGANISM: homo sapiens
(F) TISSUE TYPE: Melanoma

(ix) FEATURE:
(A) NAME/KEY: 3'UTR
(B) LOCATION:340..679

(ix) FEATURE:
(A) NAME/KEY: 5'UTR
(B) LOCATION:1..9

(ix) FEATURE:
(A) NAME/KEY: CDS
(B) LOCATION:10..339

(xi) SEQUENCE DESCRIPTION: SEQ ID NO: 1:
CGACGGGCG ATG CTG ATG GCC CAG GAG GCC CTG GCA TTC CTG ATG GCC 48
CAG GGG GCA ATG CTG GCG GCC CAG GAG AGG CGG GTG CCA CGG GCG GCA 96
GAG GTC CCC GGG GCG CAG GGG CAG CAA GGG CCT CGG GGC CGA GAG GAG 144
GCG CCC CGC GGG GTC CGC ATG GCG GTG CCG CTT CTG CGC AGG ATG GAA 192
GGT GCC CCT GCG GGG CCA GGA GGC CGG ACA GCC GCC TGC TTC AGT TGC 240
ACA TCA CGA TGC CTT TCT CGT CGC CCA TGG AAG CGG AGC TGG TCC GCA 288
GGA TCC TGT CCC GGG ATG CCG CAC CTC TCC CCC GAC CAG GGG CGG TTC 336
TGA AGGACTTCAC CGTGTCCGGC AACCTACTGT TTATCCGACT GACTGCTGCA 389
GACCACCGCC AACTGCAGCT CTCCATCAGC TCCTGTCTCC AGCAGCTTTC CCTGTTGATG 449
TGGATCACGC AGTGCTTTCT GCCCGTGTTT TTGGCTCAGG CTCCCTCAGG GCAGAGGCGC 509
TAAGCCCAGC CTGGCGCCCC TTCCTAGGTC ATGCCTCCTC CCCTAGGGAA TGGTCCCAGC 569
ACGAGTGGCC AGTTCATTGT GGGGGCCTGA TTGTTTGTCG CTGGAGGAGG ACGGCTTACA 629
TGTTTGTTTC TGTAGAAAAT AAAGCTGAGC TACGAAAAAA AAAAAAAAAA 679

(2) INFORMATION FOR SEQ ID NO: 2:

(i) SEQUENCE CHARACTERISTICS:
(A) LENGTH: 109 amino acids
(B) TYPE: amino acid
(D) TOPOLOGY: linear

(ii) MOLECULE TYPE: protein

(xi) SEQUENCE DESCRIPTION: SEQ ID NO: 2:

(2) INFORMATION FOR SEQ ID NO: 3:

(i) SEQUENCE CHARACTERISTICS:
(A) LENGTH: 767 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: NO

(iv) ANTI-SENSE: NO

(vi) ORIGINAL SOURCE:
(A) ORGANISM: homo sapiens
(F) TISSUE TYPE: Melanoma

(ix) FEATURE:
(A) NAME/KEY: CDS
(B) LOCATION:54..596

(ix) FEATURE:
(A) NAME/KEY: 3'UTR
(B) LOCATION:597..767

(ix) FEATURE:
(A) NAME/KEY: 5'UTR
(B) LOCATION:1..53

(xi) SEQUENCE DESCRIPTION: SEQ ID NO: 3:

ATCCTCGTGG GCCCTGACCT TCTCTCTGAG AGCCGGGCAG AGGCTCCGGA GCC ATG 56
Met
1

CAG GCC GAA GGC CAG GGC ACA GGG GGT TCG ACG GGC GAT GCT GAT GGC 104
Gln Ala Glu Gly Gln Gly Thr Gly Gly Ser Thr Gly Asp Ala Asp Gly
            5                   10                  15

CCA GGA GGC CCT GGC ATT CCT GAT GGC CCA GGG GGC AAT GCT GGC GGC 152
Pro Gly Gly Pro Gly Ile Pro Asp Gly Pro Gly Gly Asn Ala Gly Gly
        20                  25                  30

CCA GGA GAG GCG GGT GCC ACG GGC GGC AGA GGT CCC CGG GGC GCA GGG 200
Pro Gly Glu Ala Gly Ala Thr Gly Gly Arg Gly Pro Arg Gly Ala Gly
    35                  40                  45

GCA GCA AGG GCC TCG GGG CCG AGA GGA GGC GCC CCG CGG GGT CCG CAT 248
Ala Ala Arg Ala Ser Gly Pro Arg Gly Gly Ala Pro Arg Gly Pro His
50                  55                  60                  65

GGC GGT GCC GCT TCT GCG CAG GAT GGA AGG TGC CCC TGC GGG GCC AGG 296
Gly Gly Ala Ala Ser Ala Gln Asp Gly Arg Cys Pro Cys Gly Ala Arg
                70                  75                  80

AGG CCG GAC AGC CGC CTG CTT CAG TTG CAC ATC ACG ATG CCT TTC TCG 344
Arg Pro Asp Ser Arg Leu Leu Gln Leu His Ile Thr Met Pro Phe Ser
            85                  90                  95

TCG CCC ATG GAA GCG GAG CTG GTC CGC AGG ATC CTG TCC CGG GAT GCC 392
Ser Pro Met Glu Ala Glu Leu Val Arg Arg Ile Leu Ser Arg Asp Ala
        100                 105                 110

GCA CCT CTC CCC CGA CCA GGG GCG GTT CTG AAG GAC TTC ACC GTG TCC 440
Ala Pro Leu Pro Arg Pro Gly Ala Val Leu Lys Asp Phe Thr Val Ser
    115                 120                 125

GGC AAC CTA CTG TTT ATC CGA CTG ACT GCT GCA GAC CAC CGC CAA CTG 488
Gly Asn Leu Leu Phe Ile Arg Leu Thr Ala Ala Asp His Arg Gln Leu
130                 135                 140                 145

CAG CTC TCC ATC AGC TCC TGT CTC CAG CAG CTT TCC CTG TTG ATG TGG 536
Gln Leu Ser Ile Ser Ser Cys Leu Gln Gln Leu Ser Leu Leu Met Trp
                150                 155                 160

ATC ACG CAG TGC TTT CTG CCC GTG TTT TTG GCT CAG GCT CCC TCA GGG 584
Ile Thr Gln Cys Phe Leu Pro Val Phe Leu Ala Gln Ala Pro Ser Gly

            165                 170                 175

CAG AGG CGC TAA GCCCAGCCTG GCGCCCCTTC CTAGGTCATG CCTCCTCCCC 636
Gln Arg Arg *
        180

TAGGGAATGG TCCCAGCACG AGTGGCCAGT TCATTGTGGG GGCCTGATTG TTTGTCGCTG 696

GAGGAGGACG GCTTACATGT TTGTTTCTGT AGAAAATAAA GCTGAGCTAC GAAAAAAAAA 756

AAAAAAAAAA A 767

(2) INFORMATION FOR SEQ ID NO: 4:

(i) SEQUENCE CHARACTERISTICS:
(A) LENGTH: 180 amino acids
(B) TYPE: amino acid
(D) TOPOLOGY: linear

(ii) MOLECULE TYPE: protein

(xi) SEQUENCE DESCRIPTION: SEQ ID NO: 4:

Met Gln Ala Glu Gly Gln Gly Thr Gly Gly Ser Thr Gly Asp Ala Asp
1               5                   10                  15

Gly Pro Gly Gly Pro Gly Ile Pro Asp Gly Pro Gly Gly Asn Ala Gly
            20                  25                  30

Gly Pro Gly Glu Ala Gly Ala Thr Gly Gly Arg Gly Pro Arg Gly Ala
        35                  40                  45

Gly Ala Ala Arg Ala Ser Gly Pro Arg Gly Gly Ala Pro Arg Gly Pro
    50                  55                  60

His Gly Gly Ala Ala Ser Ala Gln Asp Gly Arg Cys Pro Cys Gly Ala
65                  70                  75                  80

Arg Arg Pro Asp Ser Arg Leu Leu Gln Leu His Ile Thr Met Pro Phe
                85                  90                  95

Ser Ser Pro Met Glu Ala Glu Leu Val Arg Arg Ile Leu Ser Arg Asp
            100                 105                 110

Ala Ala Pro Leu Pro Arg Pro Gly Ala Val Leu Lys Asp Phe Thr Val
        115                 120                 125

Ser Gly Asn Leu Leu Phe Ile Arg Leu Thr Ala Ala Asp His Arg Gln
    130                 135                 140

Leu Gln Leu Ser Ile Ser Ser Cys Leu Gln Gln Leu Ser Leu Leu Met
145                 150                 155                 160

Trp Ile Thr Gln Cys Phe Leu Pro Val Phe Leu Ala Gln Ala Pro Ser

                165                 170                 175

Gly Gln Arg Arg *
            180

(2) INFORMATION FOR SEQ ID NO: 5:

(i) SEQUENCE CHARACTERISTICS:
(A) LENGTH: 993 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: NO

(iv) ANTI-SENSE: NO

(vi) ORIGINAL SOURCE:
(A) ORGANISM: homo sapiens
(F) TISSUE TYPE: Melanoma

(ix) FEATURE:
(A) NAME/KEY: 5'UTR
(B) LOCATION:1..55

(ix) FEATURE:
(A) NAME/KEY: CDS
(B) LOCATION:56..688

(ix) FEATURE:
(A) NAME/KEY: 3'UTR
(B) LOCATION:689..993

(xi) SEQUENCE DESCRIPTION: SEQ ID NO: 5:

GCATCCTCGT GGGCCCTGAC CTTCTCTCTG AGAGCCGGGC AGAGGCTCCG GAGCC ATG 58
Met
1

CAG GCC GAA GGC CAG GGC ACA GGG GGT TCG ACG GGC GAT GCT GAT GGC 106
Gln Ala Glu Gly Gln Gly Thr Gly Gly Ser Thr Gly Asp Ala Asp Gly
            5                   10                  15

CCA GGA GGC CCT GGC ATT CCT GAT GGC CCA GGG GGC AAT GCT GGC GGC 154
Pro Gly Gly Pro Gly Ile Pro Asp Gly Pro Gly Gly Asn Ala Gly Gly
        20                  25                  30

CCA GGA GAG GCG GGT GCC ACG GGC GGC AGA GGT CCC CGG GGC GCA GGG 202
Pro Gly Glu Ala Gly Ala Thr Gly Gly Arg Gly Pro Arg Gly Ala Gly
    35                  40                  45

GCA GCA AGG GCC TCG GGG CCG AGA GGA GGC GCC CCG CGG GGT CCG CAT 250
Ala Ala Arg Ala Ser Gly Pro Arg Gly Gly Ala Pro Arg Gly Pro His
50                  55                  60                  65

GGC GGT GCC GCT TCT GCG CAG GAT GGA AGG TGC CCC TGC GGG GCC AGG 298
Gly Gly Ala Ala Ser Ala Gln Asp Gly Arg Cys Pro Cys Gly Ala Arg
                70                  75                  80

AGG CCG GAC AGC CGC CTG CTT CAG TTG CAC ATC ACG ATG CCT TTC TCG 346
Arg Pro Asp Ser Arg Leu Leu Gln Leu His Ile Thr Met Pro Phe Ser
            85                  90                  95

TCG CCC ATG GAA GCG GAG CTG GTC CGC AGG ATC CTG TCC CGG GAT GCC 394
Ser Pro Met Glu Ala Glu Leu Val Arg Arg Ile Leu Ser Arg Asp Ala
        100                 105                 110

GCA CCT CTC CCC CGA CCA GGG GCG GTT CTG AAG GAC TTC ACC GTG TCC 442
Ala Pro Leu Pro Arg Pro Gly Ala Val Leu Lys Asp Phe Thr Val Ser
    115                 120                 125

GGC AAC CTA CTG TTT ATG TCA GTT CGG GAC CAG GAC AGG GAA GGC GCT 490
Gly Asn Leu Leu Phe Met Ser Val Arg Asp Gln Asp Arg Glu Gly Ala
130                 135                 140                 145

GGG CGG ATG AGG GTG GTG GGT TGG GGG CTG GGA TCC GCC TCC CCG GAG 538*
Gly Arg Met Arg Val Val Gly Trp Gly Leu Gly Ser Ala Ser Pro Glu
                150                 155                 160

GGG CAG AAA GCT AGA GAT CTC AGA ACA CCC AAA CAC AAG GTC TCA GAA 586
Gly Gln Lys Ala Arg Asp Leu Arg Thr Pro Lys His Lys Val Ser Glu
            165                 170                 175

CAG AGA CCT GGT ACA CCA GGC CCG CCG CCA CCC GAG GGA GCC CAG GGA 634
Gln Arg Pro Gly Thr Pro Gly Pro Pro Pro Pro Glu Gly Ala Gln Gly
        180                 185                 190

GAT GGG TGC AGA GGT GTC GCC TTT AAT GTG ATG TTC TCT GCC CCT CAC 682
Asp Gly Cys Arg Gly Val Ala Phe Asn Val Met Phe Ser Ala Pro His
    195                 200                 205

ATT TAG CCGACTGACT GCTGCAGACC ACCGCCAACT GCAGCTCTCC ATCAGCTCCT 738
Ile *
210

GTCTCCAGCA GCTTTCCCTG TTGATGTGGA TCACGCAGTG CTTTCTGCCC GTGTTTTTGG 798

CTCAGGCTCC CTCAGGGCAG AGGCGCTAAG CCCAGCCTGG CGCCCCTTCC TAGGTCATGC 858

CTCCTCCCCT AGGGAATGGT CCCAGCACGA GTGGCCAGTT CATTGTGGGG GCCTGATTGT 918

TTGTCGCTGG AGGAGGACGG CTTACATGTT TGTTTCTGTA GAAAATAAAG CTGAGCTACG 978

AAAAAAAAAA AAAAA 993

(2) INFORMATION FOR SEQ ID NO: 6:

(i) SEQUENCE CHARACTERISTICS:
(A) LENGTH: 210 amino acids
(B) TYPE: amino acid
(D) TOPOLOGY: linear

(ii) MOLECULE TYPE: protein

(xi) SEQUENCE DESCRIPTION: SEQ ID NO: 6:

Met Gln Ala Glu Gly Gln Gly Thr Gly Gly Ser Thr Gly Asp Ala Asp
1               5                   10                  15

Gly Pro Gly Gly Pro Gly Ile Pro Asp Gly Pro Gly Gly Asn Ala Gly
            20                  25                  30

Gly Pro Gly Glu Ala Gly Ala Thr Gly Gly Arg Gly Pro Arg Gly Ala
        35                  40                  45

Gly Ala Ala Arg Ala Ser Gly Pro Arg Gly Gly Ala Pro Arg Gly Pro
    50                  55                  60

His Gly Gly Ala Ala Ser Ala Gln Asp Gly Arg Cys Pro Cys Gly Ala
65                  70                  75                  80

Arg Arg Pro Asp Ser Arg Leu Leu Gln Leu His Ile Thr Met Pro Phe
                85                  90                  95

Ser Ser Pro Met Glu Ala Glu Leu Val Arg Arg Ile Leu Ser Arg Asp
            100                 105                 110

Ala Ala Pro Leu Pro Arg Pro Gly Ala Val Leu Lys Asp Phe Thr Val
        115                 120                 125

Ser Gly Asn Leu Leu Phe Met Ser Val Arg Asp Gln Asp Arg Glu Gly
    130                 135                 140

Ala Gly Arg Met Arg Val Val Gly Trp Gly Leu Gly Ser Ala Ser Pro
145                 150                 155                 160

Glu Gly Gln Lys Ala Arg Asp Leu Arg Thr Pro Lys His Lys Val Ser
                165                 170                 175

Glu Gln Arg Pro Gly Thr Pro Gly Pro Pro Pro Pro Glu Gly Ala Gln
            180                 185                 190

Gly Asp Gly Cys Arg Gly Val Ala Phe Asn Val Met Phe Ser Ala Pro
        195                 200                 205

His Ile  *
    210

(2) INFORMATION FOR SEQ ID NO: 7:

(i) SEQUENCE CHARACTERISTICS:
(A) LENGTH: 752 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: NO

(iv) ANTI-SENSE: NO

(vi) ORIGINAL SOURCE:
(A) ORGANISM: Homo sapiens

(ix) FEATURE:
(A) NAME/KEY: 5'UTR
(B) LOCATION:1..53

(ix) FEATURE:
(A) NAME/KEY: CDS
(B) LOCATION:54..596

(ix) FEATURE:
(A) NAME/KEY: 3'UTR
(B) LOCATION:597..752

(xi) SEQUENCE DESCRIPTION: SEQ ID NO: 7:

ATCCTCGTGG GCCCTGACCT TCTCTCTGAG AGCCGGGCAG AGGCTCCGGA GCC ATG 56
Met
1

CAG GCC GAA GGC CGG GGC ACA GGG GGT TCG ACG GGC GAT GCT GAT GGC 104
Gln Ala Glu Gly Arg Gly Thr Gly Gly Ser Thr Gly Asp Ala Asp Gly
            5                   10                  15

CCA GGA GGC CCT GGC ATT CCT GAT GGC CCA GGG GGC AAT GCT GGC GGC 152
Pro Gly Gly Pro Gly Ile Pro Asp Gly Pro Gly Gly Asn Ala Gly Gly
        20                  25                  30

CCA GGA GAG GCG GGT GCC ACG GGC GGC AGA GGT CCC CGG GGC GCA GGG 200
Pro Gly Glu Ala Gly Ala Thr Gly Gly Arg Gly Pro Arg Gly Ala Gly
    35                  40                  45

GCA GCA AGG GCC TCG GGG CCG GGA GGA GGC GCC CCG CGG GGT CCG CAT 248
Ala Ala Arg Ala Ser Gly Pro Gly Gly Gly Ala Pro Arg Gly Pro His
50                  55                  60                  65

GGC GGC GCG GCT TCA GGG CTG AAT GGA TGC TGC AGA TGC GGG GCC AGG 296
Gly Gly Ala Ala Ser Gly Leu Asn Gly Cys Cys Arg Cys Gly Ala Arg
                70                  75                  80

GGG CCG GAG AGC CGC CTG CTT GAG TTC TAC CTC GCC ATG CCT TTC GCG 344

Gly Pro Glu Ser Arg Leu Leu Glu Phe Tyr Leu Ala Met Pro Phe Ala
            85                  90                  95

ACA CCC ATG GAA GCA GAG CTG GCC CGC AGG AGC CTG GCC CAG GAT GCC 392
Thr Pro Met Glu Ala Glu Leu Ala Arg Arg Ser Leu Ala Gln Asp Ala
        100                 105                 110

CCA CCG CTT CCC GTG CCA GGG GTG CTT CTG AAG GAG TTC ACT GTG TCC 440
Pro Pro Leu Pro Val Pro Gly Val Leu Leu Lys Glu Phe Thr Val Ser
    115                 120                 125

GGC AAC ATA CTG ACT ATC CGA CTG ACT GCT GCA GAC CAC CGC CAA CTG 488
Gly Asn Ile Leu Thr Ile Arg Leu Thr Ala Ala Asp His Arg Gln Leu
130                 135                 140                 145

CAG CTC TCC ATC AGC TCC TGT CTC CAG CAG CTT TCC CTG TTG ATG TGG 536
Gln Leu Ser Ile Ser Ser Cys Leu Gln Gln Leu Ser Leu Leu Met Trp
                150                 155                 160

ATC ACG CAG TGC TTT CTG CCC GTG TTT TTG GCT CAG CCT CCC TCA GGG 584
Ile Thr Gln Cys Phe Leu Pro Val Phe Leu Ala Gln Pro Pro Ser Gly
            165                 170                 175

CAG AGG CGC TAA GCCCAGCCTG GCGCCCCTTC CTAGGTCATG CCTCCTCCCC 636
Gln Arg Arg *
        180

TAGGGAATGG TCCCAGCACG AGTGGCCAGT TCATTGTGGG GGCCTGATTG TTTGTCGCTG 696

GAGGAGGACG GCTTACATGT TTGTTTCTGT AGAAAATAAA ACTGAGCTAC GAAAAA 752

(2) INFORMATION FOR SEQ ID NO: 8:

(i) SEQUENCE CHARACTERISTICS:
(A) LENGTH: 180 amino acids
(B) TYPE: amino acid
(D) TOPOLOGY: linear

(ii) MOLECULE TYPE: protein

(xi) SEQUENCE DESCRIPTION: SEQ ID NO: 8:

Met Gln Ala Glu Gly Arg Gly Thr Gly Gly Ser Thr Gly Asp Ala Asp
1               5                   10                  15

Gly Pro Gly Gly Pro Gly Ile Pro Asp Gly Pro Gly Gly Asn Ala Gly
            20                  25                  30

Gly Pro Gly Glu Ala Gly Ala Thr Gly Gly Arg Gly Pro Arg Gly Ala
        35                  40                  45

Gly Ala Ala Arg Ala Ser Gly Pro Gly Gly Gly Ala Pro Arg Gly Pro
    50                  55                  60

His Gly Gly Ala Ala Ser Gly Leu Asn Gly Cys Cys Arg Cys Gly Ala
65                  70                  75                  80

Arg Gly Pro Glu Ser Arg Leu Leu Glu Phe Tyr Leu Ala Met Pro Phe
                85                  90                  95

Ala Thr Pro Met Glu Ala Glu Leu Ala Arg Arg Ser Leu Ala Gln Asp
            100                 105                 110

Ala Pro Pro Leu Pro Val Pro Gly Val Leu Leu Lys Glu Phe Thr Val
        115                 120                 125

Ser Gly Asn Ile Leu Thr Ile Arg Leu Thr Ala Ala Asp His Arg Gln
    130                 135                 140

Leu Gln Leu Ser Ile Ser Ser Cys Leu Gln Gln Leu Ser Leu Leu Met
145                 150                 155                 160

Trp Ile Thr Gln Cys Phe Leu Pro Val Phe Leu Ala Gln Pro Pro Ser
                165                 170                 175

Gly Gln Arg Arg  *
            180

(2) INFORMATION FOR SEQ ID NO: 9:

(i) SEQUENCE CHARACTERISTICS:
(A) LENGTH: 752 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: NO

(iv) ANTI-SENSE: NO

(vi) ORIGINAL SOURCE:
(A) ORGANISM: homo sapiens

(ix) FEATURE:
(A) NAME/KEY: 5'UTR
(B) LOCATION:1..93

(ix) FEATURE:
(A) NAME/KEY: CDS
(B) LOCATION:94..270

(ix) FEATURE:
(A) NAME/KEY: 3'UTR
(B) LOCATION:271..752

(xi) SEQUENCE DESCRIPTION: SEQ ID NO: 9:

ATCCTCGTGG GCCCTGACCT TCTCTCTGAG AGCCGGGCAG AGGCTCCGGA GCCATGCAGG 60

CCGAAGGCCG GGGCACAGGG GGTTCGACGG GCG ATG CTG ATG GCC CAG GAG GCC 114
Met Leu Met Ala Gln Glu Ala
1               5

CTG GCA TTC CTG ATG GCC CAG GGG GCA ATG CTG GCG GCC CAG GAG AGG 162
Leu Ala Phe Leu Met Ala Gln Gly Ala Met Leu Ala Ala Gln Glu Arg
        10                  15                  20

CGG GTG CCA CGG GCG GCA GAG GTC CCC GGG GCG CAG GGG CAG CAA GGG 210
Arg Val Pro Arg Ala Ala Glu Val Pro Gly Ala Gln Gly Gln Gln Gly
    25                  30                  35

CCT CGG GGC CGG GAG GAG GCG CCC CGC GGG GTC CGC ATG GCG GCG CGG 258
Pro Arg Gly Arg Glu Glu Ala Pro Arg Gly Val Arg Met Ala Ala Arg
40                  45                  50                  55

CTT CAG GGC TGA ATGGATGCTG CAGATGCGGG GCCAGGGGGC CGGAGAGCCG 310
Leu Gln Gly *

CCTGCTTGAG TTCTACCTCG CCATGCCTTT CGCGACACCC ATGGAAGCAG AGCTGGCCCG 370

CAGGAGCCTG GCCCAGGATG CCCCACCGCT TCCCGTGCCA GGGGTGCTTC TGAAGGAGTT 430

CACTGTGTCC GGCAACATAC TGACTATCCG ACTGACTGCT GCAGACCACC GCCAACTGCA 490

GCTCTCCATC AGCTCCTGTC TCCAGCAGCT TTCCCTGTTG ATGTGGATCA CGCAGTGCTT 550

TCTGCCCGTG TTTTTGGCTC AGCCTCCCTC AGGGCAGAGG CGCTAAGCCC AGCCTGGCGC 610

CCCTTCCTAG GTCATGCCTC CTCCCCTAGG GAATGGTCCC AGCACGAGTG GCCAGTTCAT 670

TGTGGGGGCC TGATTGTTTG TCGCTGGAGG AGGACGGCTT ACATGTTTGT TTCTGTAGAA 730

AATAAAACTG AGCTACGAAA AA 752

(2) INFORMATION FOR SEQ ID NO: 10:

(i) SEQUENCE CHARACTERISTICS:
(A) LENGTH: 58 amino acids
(B) TYPE: amino acid
(D) TOPOLOGY: linear

(ii) MOLECULE TYPE: protein

(xi) SEQUENCE DESCRIPTION: SEQ ID NO: 10:

Met Leu Met Ala Gln Glu Ala Leu Ala Phe Leu Met Ala Gln Gly Ala
1               5                   10                  15

Met Leu Ala Ala Gln Glu Arg Arg Val Pro Arg Ala Ala Glu Val Pro
            20                  25                  30

Gly Ala Gln Gly Gln Gln Gly Pro Arg Gly Arg Glu Glu Ala Pro Arg
        35                  40                  45

Gly Val Arg Met Ala Ala Arg Leu Gln Gly *
    50                  55

2) INFORMATION FOR SEQ ID NO: 11:

(i) SEQUENCE CHARACTERISTICS:
(A) LENGTH: 11 amino acids
(B) TYPE: amino acid
(D) TOPOLOGY: linear

(ii) MOLECULE TYPE: protein

(xi) SEQUENCE DESCRIPTION: SEQ ID NO: 11:

Met Leu Met Ala Gln Glu Ala Leu Ala Phe Leu
1 5 10....

2) INFORMATION FOR SEQ ID NO: 12:

(i) SEQUENCE CHARACTERISTICS:
(A) LENGTH: 10 amino acids
(B) TYPE: amino acid
(D) TOPOLOGY: linear

(ii) MOLECULE TYPE: protein

(xi) SEQUENCE DESCRIPTION: SEQ ID NO: 12:

Leu Met Ala Gln Glu Ala Leu Ala Phe Leu
1 5 10.

(2) INFORMATION FOR SEQ ID NO: 13:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: synthetic DNA

GGTGACACTA TAGAAGGTAC G 21

(2) INFORMATION FOR SEQ ID NO: 14:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: synthetic DNA

TGATGTGCAA CTGAAGCAGG..........................20

(2) INFORMATION FOR SEQ ID NO: 15:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: synthetic DNA

GCACTGCGTG ATCCACATCA A 21

(2) INFORMATION FOR SEQ ID NO: 16:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: synthetic DNA

CGACTCACTA TAGGGAGAGA G 21

(2) INFORMATION FOR SEQ ID NO: 17:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: synthetic DNA

GCACATCACG ATGCCTTTCT CGTCG 25

(2) INFORMATION FOR SEQ ID NO: 18:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: synthetic DNA

CACACAAAGC TTGGCTTAGC GCCTCTGCCC TG.....................32

(2) INFORMATION FOR SEQ ID NO: 19:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: synthetic DNA

CACACAGGAT CCATGGATGC TGCAGATGCG..........................30

(2) INFORMATION FOR SEQ ID NO: 20:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: synthetic DNA

GAAGAACATA TGCTGATGGC CCAGGAGGC 29

(2) INFORMATION FOR SEQ ID NO: 21:

(i) SEQUENCE CHARACTERISTICS:
(A) LENGTH: 28 base pairs

(B) TYPE: nucleic acid
(C) STRANDEDNESS: single
(D) TOPOLOGY: linear

(ii) MOLECULE TYPE: synthetic DNA

TTAAAGATCT CAGAACCGCC CCTGGTCG 28

## Claims

1. Tumor-associated antigen encoded by the ORF-1 of LAGE-1 cDNA and by the ORF-1 of cDNAs hybridizing with LAGE-1.

2. A tumor-associated antigen of claim 1 designated CAMEL which has the amino acid sequences set forth in SEQ ID NO: 2.

3. A tumor-associated antigen of claim 1, encoded by the ORF-1 of the NY-ESO-1 cDNA, the polynucleotide sequence of which is set forth in SEQ ID NO: 7.

4. A tumor-associated antigen of any of claims 1 to 3 for use in cancer therapy.

5. An immunogenic (poly)peptide derived from a tumor-associated antigen as defined in any one of claims 1 to 3.

6. The immunogenic (poly)peptide of claim 5, characterized in that it is derived from CAMEL.

7. The immunogenic (poly)peptide of claim 6, characterized in that it has the amino acid sequence Met Leu Met Ala Gln Glu Ala Leu Ala Phe Leu (SEQ ID NO: 11).

8. The immunogenic (poly)peptide of claim 6, characterized in that it has the amino acid sequence Leu Met Ala Gln Glu Ala Leu Ala Phe Leu (SEQ ID NO: 12).

9. The immunogenic (polypeptides) of any one of claims 6 to 8 for use in cancer immunotherapy.

10. A pharmaceutical composition containing an immunogenic (poly)peptide of any one of claims 6 to 8.

11. An isolated DNA molecule comprising the ORF-1 of LAGE-1 cDNA.

12. An isolated DNA molecule comprising the ORF-1 of a cDNA hybridizing with LAGE-1 under low stringency conditions.

13. An isolated cDNA molecule encoding CAMEL.

14. The isolated cDNA molecule of claim 13 which comprises nucleotides 54 - 336 of the sequence set forth in SEQ ID NO: 1.

15. An isolated DNA molecule comprising the ORF-1 of the NY-ESO-1 cDNA, which is set forth in SEQ ID NO: 9.

16. Recombinant DNA molecule comprising a DNA molecule as defined in any one of claims 11-15.

17. A DNA molecule of any one of claims 11 to 16 for use in cancer immunotherapy.
